(19) Europäisches Patentamt
European Patent Office
Office européen des brevets

(11) **EP 0 596 809 B1**

(12) **FASCICULE DE BREVET EUROPEEN**

(45) Date de publication et mention
de la délivrance du brevet:
**11.06.2003 Bulletin 2003/24**

(51) Int Cl.7: **C08K 5/07**, C07C 45/45,
C08L 27/06

(21) Numéro de dépôt: **93402721.0**

(22) Date de dépôt: **05.11.1993**

(54) **Proédé de preparation de Beta-dicetones**

Verfahren zur Herstellung von Beta-Diketonen

Process for the preparation of Beta-diketones

(84) Etats contractants désignés:
**AT BE CH DE DK ES FR GB GR IE IT LI LU NL PT SE**

(30) Priorité: **06.11.1992 FR 9213366**
**01.02.1993 FR 9301025**

(43) Date de publication de la demande:
**11.05.1994 Bulletin 1994/19**

(60) Demande divisionnaire:
**02008807.6 / 1 229 073**

(73) Titulaire: **RHODIA CHIMIE**
**92408 Courbevoie Cédex (FR)**

(72) Inventeurs:
• **Chassaing, Serge**
**F-79500 Melle (FR)**
• **Gay, Michel**
**F-69100 Villeurbanne (FR)**
• **Mur, Gilles**
**F-94100 Saint-Maur des Fosses (FR)**

(74) Mandataire: **Wattremez, Catherine et al**
**Rhodia Services,**
**Direction de la Propriété Industrielle,**
**40, rue de la Haie-Coq**
**93306 Aubervilliers Cedex (FR)**

(56) Documents cités:
**EP-A- 0 028 758**     **EP-A- 0 062 813**
**EP-A- 0 507 013**     **FR-A- 2 165 836**
**US-A- 3 994 869**

• **CHEMICAL ABSTRACTS, vol. 92, no. 10, 10 Mars 1980, Columbus, Ohio, US; abstract no. 77434b, & JP-A-54 102 348 (ADEKA ARGUS CHEMICAL CO., LTD) 11 Aoüt 1979**
• **CHEMICAL ABSTRACTS, vol. 115, no. 10, 9 Septembre 1991, Columbus, Ohio, US; abstract no. 93760d, & JP-A-02 263 853 (ADEKA ARGUS CHEMICAL CO., LTD.) 26 Octobre 1990**
• **EUR. POLYM. J. vol. 25, no. 12 , 1989 , MARSH BARTON, GB pages 1245 - 1250 K.S. MINSKER ET AL. 'Stabilization of poly(vinyl chloride) by beta-dicarbonyl compounds'**
• **CHEMICAL ABSTRACTS, vol. 118, no. 7, 15 Février 1993, Columbus, Ohio, US; abstract no. 59367, & J. PRAKT. CHEM., CHEM. ZTG. vol. 334, no. 5 , 1992 pages 435 - 436 MICKLER, W. ET AL. 'By-products of classical beta-diketone syntheses'**
• **CHEMICAL ABSTRACTS, vol. 116, no. 26, 29 Juin 1992, Columbus, Ohio, US; abstract no. 268323p, & SEP. SCI. TECHNOL. vol. 27, no. 8-9 , 1992 pages 1171 - 1179 MICKLER., W. ET AL. 'The characterization of the active components in commercial beta-diketone-type extractants LIX 54 and MX 80 A'**

EP 0 596 809 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

**Description**

La présente invention porte sur une méthode de préparation de β-dicétones par une réaction de condensation de Claisen, qui est représentée comme suit:

$$R_4COCHR_5H + R_6C(O)OR_7 + RO^- \longrightarrow [R_4COCR_5COR_6]\text{-} + R_7OH + ROH.$$

Les β-dicétones sont une classe de composés qui bénéficient d'applications commerciales très diverses, telles que dans l'extraction de métaux et dans la stabilisation de polymères. Les β-dicétones sont aujourd'hui les meilleurs stabilisants organiques facilement disponibles pour les polymères halogénés, tels que le PVC. C'est pourquoi ces composés sont devenus de plus en plus importants sur le plan commercial.

Le coût de fabrication a été une source principale d'inquiétude dans le développement de β-dicétones, car il est tout à fait impensable , par exemple, d'utiliser des agents stabilisants coûteux dans l'industrie des polymères.

Actuellement, la méthode la plus courante qui est utilisée pour la préparation de β-dicétones est la réaction d'un ester avec un carbanion d'une cétone, qui est divulguée dans le brevet européen N°. 0.454.623 (Ciba-Geigy AG) et dans le brevet américain N°. 5.015.777 (Witco). Cette méthode peut toutefois présenter l'inconvénient de conduire à une abondance de réactions secondaires, telles que la crotonisation et la formation de β-céto-esters.

Par exemple, dans le brevet européen N°. 0.454.623, on a essayé d'améliorer le rendement en β-dicétones par l'utilisation de diméthylsulfoxyde (DMSO), un solvant coûteux, éventuellement avec l'addition d'alcool, à une faible température et en présence d'hydroxyde de sodium ou d'un alcoolate. Les rendements en ester peuvent toutefois être médiocres.

De même, dans le brevet américain N°. 5.015.777, les solvants mis en oeuvre sont moins coûteux et se séparent plus facilement du mélange réactionnel brut, mais on utilise un grand excès de l'ester. Après élimination des alkylbenzoates, le rendement par rapport à l'ester peut être médiocre à mauvais. De plus, de mauvais rendements de réaction conduisent à un mélange réactionnel qui ne peut pas être mis en oeuvre sans une purification très efficace, et le grand excès de l'ester nécessite un recyclage, ce qui est souvent coûteux et par ailleurs indésirable.

C'est pourquoi un objet de l'invention est de proposer une méthode permettant d'obtenir d'excellents rendements en β-dicétones tout en utilisant des réactifs bon marché. Un autre objet de la présente invention est de proposer une méthode permettant d'obtenir des β-dicétones de grande pureté.

En conformité avec ces objets et d'autres, la présente invention porte sur une méthode de préparation de β-dicétones de la formule

$$R_4COCHR_5COR_6$$

comprenant la réaction d'une cétone de formule $R_4\text{-}CO\text{-}CHR_5H$ avec un ester de formule $R_6\text{-}C(O)\text{-}OR_7$, en présence d'un alcoolate dont l'alcool est volatil dans les conditions opératoires, dans laquelle ladite cétone est ajoutée audit ester tout en éliminant l'alcool progressivement au fur et à mesure de sa formation,

dans lesquelles :

- $R_4$ et $R_6$, identiques ou différents, représentent chacun un groupement hydrocarboné et peuvent éventuellement être reliés pour former un composé cyclique ;
- $R_5$ est un hydrogène ou un groupement hydrocarboné ;
- $R_7$ est un groupement hydrocarboné tel que l'alcool $R_7OH$ soit volatil dans les conditions réactionnelles ;

et dans laquelle le rapport molaire de ladite cétone audit ester est dans la gamme de 2/3 :1 à 1:1.

De préférence, $R_4$ et $R_6$ sont un groupement hydrocarboné ayant de 1 à 30 atomes de carbone, plus préférentiellement de 1 à 18 atomes de carbone. De façon tout à fait préférentielle, $R_4$ et $R_6$ sont choisis parmi les radicaux alkyle ou alkényle, linéaires ou ramifiés, ayant de 1 à 24 atomes de carbone; aralkyle ayant de 7 à 10 atomes de carbone; et aryle ou cycloaliphatique, pouvant avoir une ou plusieurs liaisons doubles ayant chacun moins de 14 atomes de carbone. $R_4$ et $R_6$ peuvent être éventuellement substitués, par exemple, par des atomes d'halogène ou des groupements alkyle, tels que méthyle et éthyle, et peuvent contenir un ou plusieurs de -O-, -CO-O-, -CO-, et Si dans la chaîne aliphatique. De manière alternative, $R_4$ et $R_6$ peuvent ensemble former un groupement divalent ayant de 2 à 5 atomes de carbone, contenant éventuellement un hétéroatome tel que l'oxygène ou l'azote.

De préférence, $R_5$ est un atome d'hydrogène ou un groupement alkyle ayant de 1 à 4 atomes de carbone, pouvant être substitués ou non substitués et pouvant contenir un ou plusieurs de -O-, -CO-O-, et -CO-. Plus préférentiellement, $R_5$ est un atome d'hydrogène.

De préférence, $R_7$ est un groupement alkyle ayant de 1 à 4 atomes de carbone. Plus préférentiellement, $R_7$ est un groupement méthyle.

Selon un mode de réalisation préféré de la présente invention, le mélange réactionnel contient l'alcoolate, l'ester et un solvant. A ce mélange, on ajoute la cétone, tout en éliminant progressivement tout alcool qui est formé. La cétone peut être ajoutée à l'état pur ou en solution, seule ou avec une proportion de l'ester qui n'est pas dans le mélange de départ.

L'ester peut être mis en oeuvre à l'état simple ou en autocondensat partiel ou total. Selon un mode de réalisation de l'invention, si l'ester a au moins un hydrogène dans la position alpha du groupement ester, il peut être mis en oeuvre au moins partiellement sous forme de $\beta$-céto-ester qui en est dérivé, ou sous la forme d'un de ses sels. Par exemple, si la formule de $R_6$ est écrite comme

$$(R'_6)(R''_6)CH-$$

le $\beta$-céto-ester est alors écrit comme

$$R_6C(O)(R'_6)(R''_6)CC(O)OR_7,$$

où $R'_6$ et $R''_6$ peuvent être chacun hydrogène ou un groupement hydrocarboné. Ce résultat inattendu contredit totalement l'enseignement de l'art pertinent et permet l'utilisation de conditions favorisant la formation de $\beta$-céto-ester, dans la mesure où l'addition de la cétone dans les bonnes conditions opératoires donne la(les) $\beta$-dicétone(s) recherchée(s).

L'alcoolate peut être n'importe quel alcoolate dont l'alcool est volatil dans les conditions réactionnelles, et est de préférence un alcoolate de métal alcalin ou alcalino-terreux. Plus préférentiellement, l'alcoolate est un alcoxyde de métal alcalin ou alcalino-terreux. Les alcoolates particulièrement préférés sont les alcoxydes de métaux alcalins ayant de 1 à 4 atomes de carbone. De façon tout à fait préférentielle, l'alcoolate est le méthylate de sodium (méthoxyde).

Pendant l'addition de la cétone, tout alcool formé est éliminé, de préférence par distillation en portant le solvant au reflux. C'est pourquoi la température réactionnelle est de préférence au moins supérieure de 20°C au point d'ébullition de l'alcool. Plus préférentiellement, la température réactionnelle est au moins 70°C, de façon tout à fait préférentielle au moin 100°C. Dans un mode de réalisation préféré particulier de la présente invention, la température réactionnelle varie de 100 à 200°C, et plus préférentiellement de 110 à 150°C, sous presssion atmosphérique.

La pression n'est pas critique pour la présente méthode, sauf dans la mesure où la sélection d'une pression particulière peut faciliter l'élimination rapide des alcools libérés dans le milieu réactionnel. Si la réaction se produit sous pression réduite, la température réactionnelle préférée varie de 80 à 150°C.

De préférence, le rapport molaire de la cétone à l'ester est inférieur à 1, plus préférentiellement compris entre 2/3 et 1. En général, un excès de l'ester de 10 à 20 % est utilisé par rapport à la cétone.

De préférence, le rapport molaire stoechiométrique de la cétone à l'alcoolate est inférieur à 1, plus préférentiellement compris entre 2/3 et 1. En général, un excès de l'alcoolate de 5 à 25 % est utilisé par rapport à la cétone.

Le solvant, qui peut être une substance pure ou un mélange, est de préférence choisi pour avoir un point d'ébullition élevé à pression atmosphérique de telle sorte que l'alcool libéré par les diverses réactions puisse être éliminé sans élimination du solvant. C'est pourquoi on préfère qu'à pression atmosphérique, le solvant ait un point d'ébullition variant de 100 à 250°C, plus préférentiellement de 110 à 200°C. De façon tout à fait préférentielle, le solvant a un point d'ébullition de 130 à 200°C.

Pour une bonne séparation du solvant de l'alcool, le point d'ébullition du solvant est de préférence au moins 20°C au-dessus de celui de l'alcool, plus préférentiellement au moins 40°C, et de façon tout à fait préférentielle au moins 60°C au-dessus. De manière alternative, le solvant peut contenir, comme composant mineur, une substance qui forme un minimum d'azéotrope avec l'alcool; on préfère que le point d'ébullition de cet azéotrope soit suffisamment distant de celui du mélange réactionnel de telle sorte qu'il (l'azéotrope) soit facilement séparé du solvant dans le reflux.

Les mêmes contraintes qui s'appliquent à l'alcool de l'alcoolate s'appliquent à l'alcool de l'ester, mutatis mutandis. Ainsi, avec l'alcool de l'ester, le solvant a également de préférence un point d'ébullition d'au moins 20°C au-dessus de celui de l'alcool $R_7OH$, plus préférentiellement au moins 40°C au-dessus, et de façon tout à fait préférentielle au moins 60°C au-dessus.

Si les solvants qui sont mis en oeuvre bouillent à une température supérieure à celle à laquelle on désire conduire la réaction, l'élimination des alcools alcools peut être facilitée par toute technique appropriée connue dans l'art, telle que le barbotage d'un gaz inerte ou travailler sous pression réduite.

Si le solvant est constitué d'un seul constituant principal, on préfère choisir un solvant qui ne forme pas un azéotrope avec l'alcool formé, et éviter ainsi une situation où l'alcool est réintroduit dans le mélange réactionnel lorsque l'azéotrope est soumis au reflux. Dans le cas contraire, il est possible d'éliminer progressivement l'azéotrope contenant l'alcool au fur et à mesure de sa libération.

Le taux d'addition de la cétone est choisi de telle sorte que la teneur en alcool dans le mélange réactionnel soit maintenue aussi faible que possible, de préférence de telle sorte que pendant l'addition, le rapport entre l'alcoolate et l'alcool libre en solution soit de préférence au moins d'environ $10^3$:1, plus préférentiellement $10^6$:1, de façon tout à fait préférentielle $10^9$:1. Si la température réactionnelle est inférieure à 70°C, il est préférable de réduire le taux d'addition en divisant, par exemple, par un facteur de 2.

Des modes de réalisation particulièrement préférés de la présente méthode sont un taux d'addition d'au moins 0,001 mole de cétone par seconde par litre de suspension d'alcoolate dans le mélange réactionnel de départ, qui peut donner de bons rendements, et un taux d'au plus 0,0002 mole/seconde/litre qui peut donner d'excellents résulats.

Le procédé selon l'invention permet de préparer notamment des $\beta$-dicetones qui pourront constituer une composition stabilisante pour les polymères halogénés, liquide à la température d'utilisation de la composition, au moins partiellement miscible avec d'autres ingrédients d'une formulation stabilisante liquide "tout-en-un".

Plus particulièrement, cette composition stabilisante pour les polymères halogénés comprenant une quantité efficace d'au moins un composé $\beta$-dicétone représenté par la formule (I):

$$R_1COCH_2COR_2 \tag{I}$$

ou la formule (II)

$$R_2COCH_2COR_2 \tag{II}$$

dans lesquelles $R_1$ est représenté par la formule

$$(Y)_n\text{-}\Phi\text{-},$$

dans laquelle $\Phi$ est phényle et chaque Y, identique ou différent, est un atome d'hydrogène ou un groupement choisi parmi les chaînes hydrocarbonées ayant de 1 à 12 atomes de carbone, les alcoxy, les silyle et les atomes d'halogène non réactifs; chaque $R_2$, identique ou différent, représente un atome d'hydrogène ou un groupement choisi parmi les chaînes hydrocarbonées ayant de 1 ou de 5 à 12 atomes de carbone, éventuellement interrompus par un ou plusieurs atomes d'oxygène, des aralkyle, des alcoxy et des silyle; et n représente un nombre entier de 0 à 3; à condition que si le nombre d'atomes de carbone dans $R_2$ de la formule (I) est inférieur à 5, la somme des carbones contenus dans Y est au moins 3 et au plus 12, et que dans la formule (II), le nombre total d'atomes de carbone dans les deux $R_2$ est au moins 10.

De préférence, Y est un groupement alkyle ayant de 1 à 12 atomes de carbone. De préférence, la somme du nombre d'atomes de carbone dans les groupements Y est inférieure à 6. Plus préférentiellement, la somme du nombre d'atomes de carbone est 3. Avantageusement, la somme du nombre d'atomes de carbone dans les groupements radicaux Y et de ceux dans $R_2$ est inférieure à 12, de préférence 10.

Il est également préférable que l'un des Y, ou un seul Y, soit en position para par rapport à la chaîne $\beta$-dicétone.

De préférence, si la chaîne hydrocarbonée est linéaire, le nombre d'atomes de carbone dans $R_2$ est de préférence compris entre 5 et 9, plus préférentiellement entre 5 et 7. Si la chaîne hydrocarbonée est ramifiée, le nombre d'atomes de carbone dans $R_2$ est de préférence compris entre 5 et 12, plus préférentiellement entre 5 et 9. De préférence, si la chaîne hydrocarbonée est interrompue par plus d'un atome d'oxygène, chaque atome d'oxygène est de préférence séparé de l'autre par au moins 2 atomes de carbone, comme dans les glymes.

La quantité de composés $\beta$-dicétone à mettre en oeuvre dans la composition stabilisante peut être facilement déterminée par l'homme de l'art. De préférence, la composition stabilisante comprend (sur base molaire) au moins les 2/3 des composés $\beta$-dicétone, plus préférentiellement les 3/4, et de façon tout à fait préférentielle les 4/5. De préférence, le constituant majeur de la composition stabilisante est la $\beta$-dicétone représentée par la formule (I).

On préfère, outre le composé $\beta$-dicétone représenté par la formule (I) ou (II), qui est le constituant majeur de la composition stabilisantè, que la composition comprenne encore au moins un autre composé $\beta$-dicétone de formule (I), de formule (II), et/ou de formule (III)

$$R_1COCH_2COR_1 \tag{III}$$

dans laquelle chaque $R_1$, identique ou différent, a la même signification que décrit précédemment.

On préfère particulièrement que la quantité totale du au moins un autre composé β-dicétone de formules (I), (II), et/ou (III), ajoutée au polymère en plus du constituant majeur, soit au moins 5 % de la quantité du constituant majeur. Plus préférentiellement, la quantité totale des autres composés β-dicétone est au moins 10 % de la quantité du constituant majeur, de façon tout à fait préférentielle au moins 15 %. Les quantités de chacun des autres composés β-dicétone peuvent être déterminées par le spécialiste. De préférence, la somme des quantités des composés de formule (III) n'est pas supérieure à 10 % de la somme dès quantités des composés de formules (I) et (II).

En général, la toxicité augmente ou diminue de façon linéaire en fonction du nombre d'atomes de carbone dans le composé. De manière inattendue, la toxicité des compositions β-dicétone de l'invention est de manière étonnante faible et peut être trop faible pour être déterminée. Certaines β-dicétones, qui ne font pas partie de la présente invention, présentent une toxicité qui est non négligeable mais faible. Par exemple, l'isovalerylbenzoylméthane présente une toxicité aiguë de $DL_{50}$ per os chez le rat de 4885 mg/kg. Par contre, la toxicité des composés β-dicétone selon la présente invention peut être si faible qu'elle ne peut pas être mesurée. Pour les composés dans lesquels $R_1$ est phényl et $R_2$ est un groupement linéaire, en particulier en $C_7$, la $DL_{50}$ est généralement supérieure à 5000 mg/kg (rat, p.o.).

En outre, dans les conditions opératoires dans le PVC, les compositions β-dicétone selon la présente invention peuvent ne dégager essentiellement aucune odeur, par opposition nette aux composés très proches. Ceci est en effet tout à fait remarquable car certains homologues inférieurs, tels que le produit dans lequel $R_1$ est phényle et $R_2$ est en $C_4$ ($R_2$=$(CH_3)_2CHCH_2$-), présentent une odeur intolérable lorsqu'une dégradation se produit au cours du stockage de la composition ou au cours de la transformation du PVC.

De plus, contrairement à l'opinion répandue chez l'homme de l'art que la présence, à titre de stabilisant, de β-dicétones di-aromatiques, telles que le dibenzoylméthane, se traduit par une coloration intense en présente du fer (due vraisemblablement à la forte absorption d'un chélate dans la gamme de longueur d'ondes de 400-450 nm), les compositions selon la présente invention peuvent contenir des proportions relativement élevées de dicétones di-aromatiques sans présenter d'intensification de coloration.

La β-dicétone mise en oeuvre dans la composition stabilisante doit être de préférence liquide à une température de 50°C. Plus préférentiellement, la β-dicétone est liquide à une température de 30°C; de façon tout à fait préférentielle à 0°C. Les β-dicétones particulièrement préférées sont celles qui sont liquides à température ambiante.

Lorsqu'elle est mise en oeuvre comme additif liquide homogène pour le PVC, la composition stabilisante comprenant un composé β-dicétone de formule (I) et/ou (II) décrite ci-dessus, de préférence comprend encore au moins un composé accessoire choisi parmi, entre autres, les sels du zinc, les sels des métaux alcalino-terreux, et les phosphites organiques. L'homme de l'art peut facilement choisir des composés accessoires appropriés en vue de les inclure dans la composition stabilisante de l'invention. Des exemples de tels composés incluent le propionate de zinc, le 2-éthylhexanoate de zinc, le laurate de zinc, le stéarate de zinc, l'oléate de zinc, le ricinoléate de zinc, le docosanoate de zinc, le benzoate de zinc, le para-tertiobutylbenzoate de zinc, le salicylate de zinc, le mono(2-éthylhexyl)maléate de zinc, les nonylphenates de zinc, et les sels du calcium et du magnésium baryum avec les acides maléique, acétique, diacétique, propionique, hexanoïque, 2-éthylhexanoïque, décanoïque, undécanoïque, laurique, myristique, palmitique, stéarique, oléique, ricinoléique, béhénique (docosanoïque), hydroxystéarique, hydroxyundécanoïque, benzoïque, phénylacétique, para-tertiobutylbenzoïque et salicylique, et les phénolates de calcium et de magnésium provenant du phénol et des phénols substitués par un ou plusieurs radicaux alkyle, tels que les nonylphénols.

De préférence, le rapport entre la somme des composés β-dicétone et la quantité dudit composé accessoire est d'au moins 1:100. Plus préférentiellement, le rapport est de 1:50; de façon tout à fait préférentielle de 1:20.

Les exemples suivants sont destinés simplement à illustrer l'invention et ne doivent pas être considérés comme limitants. L'homme de l'art peut faire, sans trop d'expérimentation, diverses substitutions et variations et par des moyens équivalents, procédant essentiellement de la même manière, obtenir eessentiellement les mêmes résultats sans s'écarter de l'enseignement et de l'esprit de l'invention.

Exemple 1: Synthèse d'heptanoylbenzoylméthane

Dans un ballon en pyrex de 2000 ml on a chargé:

. du méthylate de sodium, préparé extemporanément     62,6 g (1,16 mole)
. du xylène      715 ml.

Le mélange a été chauffé à 100°C, puis on a ajouté sur une période d'environ 10 min:

. du méthylheptanoate pur     158,4 g (1,10 mole).

Le mélange réactionnel a été porté au reflux (environ 137°C), puis on a ajouté progressivement sur 2 h sous agitation:

. de l'acétophénone pure      120,0 g (1,0 mole).

Pendant toutes ces étapes, le mélange réactionnel a été maintenu sous atmosphère d'azote inerte, et un mélange xylène/méthanol a été éliminé par distillation de telle sorte que la température T (°C) des vapeurs éliminées par distillation en tête de la colonne qui était munie d'un couvercle maille "multimaille" variait dans l'intervalle de 108-115°C. Le mélange distillé a été récupéré dans un ballon récepteur.

La quantité de solvant dans le milieu réactionnel a été maintenue généralement constante à environ 715 ml par addition supplémentaire de xylène.

Le milieu réactionnel est resté fluide et homogène pendant toute la durée du procédé.

A la fin de l'addition de l'acétophénone, le reflux a été poursuivi pendant 1 h, au cours duquel une petite quantité supplémentaire de méthanol a été éliminée par distillation. Pendant l'ensemble de l'opération, le mélange distillé comprenait 140 g de xylène et 62 g de méthanol. On a laissé le mélange se refroidir, puis il a été transféré sous agitation dans un bécher de 2000 ml contenant 800 g de glace et 38,6 ml d'acide sulfurique à 95 % (comprenant environ 0,7 mole d'$H_2SO_4$). La phase aqueuse a été transférée par décantation dans une ampoule à décanter, puis a été lavée avec $2 \times 100$ ml de xylène. Le xylène de lavage a ensuite été réuni avec la phase organique, et on a lavé avec $2 \times 250$ ml d'eau, séché sur du sulfate de sodium anhydre, et filtré.

On a ainsi obtenu 1014 g d'une solution dans du xylène, dans laquelle on a déterminé une teneur de 0,870 mole en β-dicétone par potentiométrie; celle-ci a représenté un rendement chimique en β-dicétone de 87 %, sur la base de la cétone chargée. On a également déterminé 0,170 mole d'acide carboxylique par potentiométrie.

Après extraction des acides carboxyliques par une quantité stoechiométrique d'hydroxyde de sodium (ou carbonate de sodium) aqueux, et élimination du xylène sous pression réduite, on a obtenu 217 g d'un produit brut sous forme d'une huile homogène.

L'analyse par chromatographie en phase gazeuse (GC) de ce produit a mis en évidence qu'il contenait trois β-dicétones (utilisables en mélange dans la stabilisation de polymères halogénés), comme suit:

-- Heptanoylbenzoylméthane      182 g (83,9 %)
-- Dibenzoylméthane      11,2 g (5,1 %)
-- Diheptanoylméthane      8,6 g (4,0 %).

Cette analyse confirme le rendement chimique global de 87 % (basé sur la cétone chargée).

La distillation de ce mélange réactionnel était facile à réaliser et a conduit (avec un rendement d'environ 96 %) à un mélange des trois β-dicétones présentant une pureté d'environ 99 %.

La distillation a également été mise en oeuvre pour isoler l'heptanoylméthane pur, avec un bon rendement (environ 90 %). Ce produit se présentait sous la forme d'une huile incolore avec un point d'ébullition de 112-115°C (à 100 Pa). Son point de fusion était de 14°C, son indice de réfraction à 30°C était de 1,5558, et sa densité à 30°C était de 1,004. L'analyse par RMN a mis en évidence qu'il comprenait 90 % de la forme énolique et 10 % de la forme dicétone.

Exemple 2: Heptanoylbenzoylméthane

On a procédé comme à l'exemple 1, la seule différence étant qu'on a ajouté seulement 144 g (1 mole) de méthylheptanoate.

L'analyse potentiométrique et l'analyse par chromatographie en phase gazeuse (CPG) de la solution finale dans du xylène a mis en évidence que le rendement en β-dicétones était de 81,1 % (basé sur la cétone chargée).

Exemple 3: Heptanoylbenzoylméthane (exemple de comparaison)

On a procédé comme à l'exemple 1, la seule différence étant qu'on a ajouté 316,8 g (2,2 moles) de méthyl heptanoate.

L'analyse potentiométrique et l'analyse par GC de la solution finale dans du xylène a mis en évidence que le rendement en β-dicétones était de 94,8 % (basé sur la cétone chargée).

Les mêmes analyses ont mis en évidence qu'il est resté seulement 0,9 mole de l'ester non consommé; ainsi, le rendement en β-dicétones, basé sur l'ester consommé, c'est-à-dire 72,9 %. était sensiblement inférieur à celui obtenu à l'exemple 5, c'est-à-dire 79,1 % (en supposant que tout l'ester à l'exemple 1 a été consommé).

Ainsi, la mise en oeuvre d'un grand excès de l'ester présente deux inconvénients majeurs:

-- l'ester est recyclé; et

-- le rendement en β-dicétones (basé sur la quantité d'ester consommé) est sensiblement inférieur (ce qui peut être expliqué par la formation de sous-produits).

Exemple 4: Octanoylbenzoylméthane

On charge dans le réacteur décrit à l'exemple 1:

.   du méthylate de sodium préparé extemporanément        62,6 g (1,16 mole)
.   du xylène        715 ml.

    du mélange a été chauffé à 100°C sous atmosphère d'azote sec, puis on a ajouté pendant une durée d'environ 10 min:

.   du méthyloctanoate pur        173,8 g (1,10 mole).

Le mélange réactionnel a été porté au reflux (environ 137°C), puis on a ajouté progressivement sur 2 h sous agitation :

.   de l'acétophénone pure        120,0 g (1,0 mole).

Les étapes ultérieures ont été les mêmes que celles décrites à l'exemple 2.
Le mélange de xylène et de méthanol qui a été éliminé par distillation pesait 203 g (comprenait environ 140 g de xylène et environ 63 g de méthanol).
1033,3 g d'une solution dans du xylène ont été éliminés, dans laquelle on a déterminé par potentiométrie 0,871 mole de β-dicétones (représentant un rendement chimique en β-dicétones de 87,1 % par rapport à la cétone chargée). 0,170 mole d'acides carboxylique a également été déterminé par potentiométrie.
Après extraction des acides carboxyliques par une quantité stochiométrique d'hydroxyde de sodium (ou de carbonate de sodium) aqueux, et élimination du xylène sous pression réduite, on a obtenu 228 g d'un produit brut sous la forme d'une huile jaune qui était fluide et homogène.
L'analyse par chromatographie en phase gazeuse (CPG) de ce produit a mis en évidence qu'il contenait trois β-dicétones, comme suit:

-- octanoylbenzoylméthane        190,9 g (83,7 %)
-- dibenzoylméthane        12,4 g (5,4 %)
-- dioctanoylméthane        10,8 g (4,7 %).

Cette analyse confirme le rendement chimique global de 87 % (basé sur la cétone chargée).
La distillation de ce mélange réactionnel était facile à réaliser et a conduit à la séparation des β-dicétones (environ 94 %) des goudrons (environ 6 %). Le rendement de la distillation était de 97 %, et le produit obtenu analysé pour les β-dicétones en quantité > 99 %.
L'isolement de l'octanoylbenzoylméthane pur a été réalisé avec un bon rendement (environ 85 %) par distillation avec précaution. Ce produit a été obtenu sous la forme d'un solide blanc avec un point de fusion bas (environ 34°C) et un point d'ébullition de 121-122°C (à 0,1 Torr). Son indice de réfraction (30°C) était de 1,5445, et sa densité à 30°C était de 0,982. L'analyse par RMN a mis en évidence qu'il comprenait 91 % de la forme énolique et 9 % de la forme dicétone.

Exemple 5: Octanoylbenzoylméthane

On a chargé dans le réacteur décrit à l'exemple 1:

.   du méthylate de sodium, préparé extemporanément        62,6 g (1,16 mole)

.   du xylène sec        150 ml.

Le mélange a été chauffé à 100°C sous atmosphère d'azote sec, puis on a ajouté sur une période d'environ 10 min:

. du méthyloctanoate, contenant 6,8 % de méthylhexanoate      189,9 g (1,20 mole).

Le mélange réactionnel a été porté au reflux (environ 137°C), puis on a ajouté progressivement sur 2 h sous agitation:

. de l'acétophénone pure      120,0 g. (1,0 mole).

Les étapes ultérieures étaient les mêmes que celles décrites à l'exemple 5, excepté que le mélange réactionnel a été acidifié avec 69 g d'$H_2SO_4$ (0,67 mole) et lavé avec 100 ml de xylène.

L'analyse par chromatographie en phase gazeuse (GC) de ce produit, fondant à environ 24°C, a mis en évidence qu'il contenait 5 β-dicétones, comme suit:

-- octanoylbenzoylméthane      80 %
-- dibenzoylméthane      6 %
-- dioctanoylméthane      4 %
-- Hexanoylbenzoylméthane      6 %
-- dihexanoylméthane      0,3 %.

La composition contenait également 3,7 % d'impuretés.

Exemples 6-12:

On a procédé comme à l'exemple 4, mais certains paramètres de la réaction ont été changés, c'est-à-dire:

-- les rapports des réactants (ester en excès, et MeONa en excès); et
-- la durée de la période d'addition de l'acétophénone.

Les résultats obtenus (rendement chimique global en β-dicétones) sont donnés au tableau 1 (par comparaison avec l'exemple 4).

Tableau 1

| Paramètres des exemples modifiés: | | | | |
|---|---|---|---|---|
| Nombre: | Durée d'addition de $\Phi COCH_3$: | Ester porté | MeONa porté | Rendement en β-dicétones (basé sur $\Phi COCH_3$): |
| 8 | 2 h | 1,1 mole | 1,16 mole | 87,1 % |
| 10 | 2 h | 1,0 mole | 1,16 mole | 80,0 % |
| 11 | 2 h | 1,175 mole | 1,16 mole | 88,7 % |
| 12 | 7 h | 1,175 mole | 1,16 mole | 88,4 % |
| 13 | 1 h | 1,175 mole | 1,16 mole | 82,7 % |
| 14 | 2 h | 1,175 mole | 1,025 mole | 88,5 % |
| 15 | 2 h | 1,175 mole | 1,075 mole | 89,5 % |
| 16 | 2 h | 1,22 mole | 1,16 mole | 88,9 % |

Ces résultats démontrent les points suivants:

-- L'effet de l'ester en excès est très marqué lorsque l'excès passe de 0 % à 10 %.
-- L'effet du méthylate en excès est très faible.
-- L'effet de la durée d'addition: des durées d'addition de 2 h et 7 h donnent essentiellement le même résultat. Toutefois, une addition trop rapide (durée de 1 h) peut avoir un effet néfaste sensible sur le résultat; on attribue ceci à l'insuffisance de la rapidité d'élimination du méthanol par rapport au taux de formation du méthanol. (Il est très important que le rapport de MeOH au MeO soit > 1,000.)

Exemple 13: Synthèse du méthyloctanoyloctanoate

On a chargé dans le réacteur décrit à l'exemple 1:

. du méthylate de sodium préparé extemporanément     54,0 g (1,0 mole)

. du xylène    715 ml.

Le mélange a été chauffé à 100°C, puis on a ajouté sur une période d'environ 10 min:

. du méthyloctanoate pur    316,0 g (2,0 moles).

Le mélange réactionnel a été porté au reflux (environ 140°C), le méthanol étant éliminé par distillation progressivement au fur et à mesure de sa formation.

On a trouvé que le méthanol se formait lentement.

Après 7,5 h de reflux et d'élimination continue du méthanol, la quantité de méthanol qui a été éliminée par distillation était d'environ 46 g, dans un mélange avec environ 10 g de xylène. L'essai est arrêté.

Après acidification du mélange réactionnel comme à l'exemple 1, 1006,8 g d'une solution jaune limpide a été obtenue dans le xylène, dans laquelle on a déterminé 0,16 mole d'acide carboxylique par potentiométrie. Après élimination de ces acides carboxyliques par la quantité stoechiométrique nécessaire d'hydroxyde de sodium (ou de carbonate de sodium) aqueux, on a déterminé 0,520 mole d'ester résiduel par CPG (le TT de l'ester était de 74 %). Après élimination du xylène sous pression réduite, on a obtenu 206,3 g d'un produit brut sous la forme d'un fluide homogène huileux avec une légère coloration jaune.

La distillation de ce produit brut a conduit à 135 g d'un produit pur présentant un point d'ébullition de 123-124°C (à 40 Pa). Ce produit était sous la forme d'une huile fluide incolore.

Les analyses et les spectres IR et RMN et SM étaient conformes à la structure du produit β-céto-ester suivant, avec une pureté >99 %:

$$c_7H_{15}COC(C_6H_{13})HC(O)OMe \ (M = 284)$$

Le rendement de β-céto-ester distillé pur (0,475 mole) (basé sur la quantité d'ester transformé, 1,48 mole) était de 64 %.

Exemple 14: Octanoylbenzoylméthane à partir du β-céto-ester

L'appareillage était le même que celui décrit à l'exemple 1, mais la dimension du réacteur n'était que de 1 L.

On a chargé dans le réacteur:

. du méthylate de sodium préparé extemporanément     43,2 g (0,8 mole)

. du xylène    475 ml.

Le mélange a été chauffé à 140°C, puis on a ajouté lentement le β-céto-ester suivant, tout en éliminant avec précaution et complètement le méthanol formé:

. du méthyloctanoyloctanoate    113,8 g (0,4 mole).

La durée d'addition était de 1 h 20. A la fin de l'addition, on a trouvé que le β-céto-ester était quantitativement sous la forme de l'énolate de sodium.

La température a été maintenue à 140°C, et on a ajouté progressivement sur une période de 2 h, tout en éliminant avec précaution et complètement le méthanol formé:

. de l'acétophénone    80,5 g (0,67 mole).

A la fin de l'addition de l'acétophénone, le mélange a été maintenu pendant encore 30 min à 140°C. A ce stade, un mélange de xylène et de méthanol pesant environ 140 g avait été éliminé par distillation comprenant environ 112 g de xylène et environ 28 g de méthanol.

Remarque: Comme à l'exemple 1, toutes les étapes ont été effectuées sous atmosphère d'azote inerte, et du xylène pour ajustement a été ajouté pour maintenir une quantité globale de solvant essentiellement constante dans le mélange réactionnel.

A la fin de cette opération, le mélange réactionnel a été refroidi et transféré sous agitation dans un bécher contenant 500 g de glace et 28 ml d'acide sulfurique à 95 % (comprenant environ 0,7 mole d'$H_2SO_4$). La phase organique a été alors séparée de la phase aqueuse comme à l'exemple 1.

675,5 g d'une solution dans du xylène a été récupérée, dans laquelle on a déterminé 0,583 mole de fonction β-dicétone par potentiométrie pour un rendement chimique en β-dicétones de 87 % (basé sur la cétone chargée). (Le β-céto-ester, trop peu acide, n'a pas gêné la détermination potentiométrique dans le milieu aqueux). On a également déterminé par potentiométrie 0,0965 mole d'acide carboxylique. On a déterminé dans le mélange réactionnel, par analyse CPG :

-- le β-céto-ester résiduel        0,011 mole (soit 2,8 %, basé sur le β-céto-ester chargé.)
-- le méthyloctanoate        0,037 mole (soit 4,6 %, basé sur le β-céto-ester chargé.)

On a également déterminé que le mélange de β-dicétone avait les composants suivants:

-- de l'octanoylbenzoylméthane        0,546 mole (soit 81,5 %, basé sur la cétone chargée.)
-- du dibenzoylméthane        0,22 mole (soit 3,3 %, basé sur la cétone chargée.)
-- du dioctanoylméthane         0,015 mole (soit 2,2 %, basé sur la cétone chargée.)

Après l'élimination du solvant, un tel mélange réactionnel pourrait être utilisé directement pour stabiliser les polymères halogénés tels que le chlorure de polyvinyle PVC).

Exemple 15: Synthèse de dibenzoylméthane

Dans un réacteur cylindrique de 2000 ml équipé

-- d'un agitateur muni de pales à racler et
-- d'un système de distillation tel que décrit à l'exemple 1, on a chargé:

. du méthylate de sodium préparé extemporanément        67,5 g (1,15 mole)
. du xylène        800ml.

Le mélange a été chauffé à 135°C, puis on a ajouté rapidement (sur une période de 5 min):

. du méthylbenzoate (98,5 %)        151,8 g (1,10 mole)

Le milieu a été porté au reflux, puis on a ajouté sur une période de 4 h, en maintenant la température de 135°C:

. de l'acétophénone        120 g (1 mole).

Le milieu réactionnel a été maintenu au reflux pendant 30 min après la fin de l'addition de l'acétophénone, et on a poursuivi la récupération du distillat (température de la vapeur 70°C). 130 ml de distillat ont été récupérés au total.
Le milieu était épais mais il demeurait susceptible d'être agité.
Ensuite le milieu a été neutralisé par une solution d'acide sulfurique à 10 %. Après les opérations de décantation et lavage, 881 g d'une solution de dibenzoylméthane dans du xylène ont été obtenus que l'on a analysés à 1,057 mole/kg pour un rendement de 93,1 % (basé sur la cétone).

Exemple 16: Synthèse de stéaroylbenzoylméthane et de palmitoylbenzoylméthane

On a chargé dans un appareil analogue à celui des exemples précédents:

. du méthylate de sodium préparé extemporanément        67,5 g (1,15 mole)
. du toluène        1000 ml.

Le mélange a été chauffé à 105°C, puis on ajouté rapidement:

. du méthylstéarate technique (contenant d'autres esters d'acides gras, en particulier le méthylpalmitate à raison de 33 %)        293 g (1,03 mole).

Le milieu a été porté au reflux, puis on a introduit sur une période de 2 h:

. de l'acétophénone        120 g (1,0 mole)

On a laissé le mélange sous reflux pendant 30 min après l'addition de l'acétophénone. Pendant toute la réaction, l'azéotrope toluène-méthanol contenant beaucoup de toluène a été récupéré en continu (température de vapeur 90°C); le volume récupéré était de 390 ml. Le milieu était homogène à la fin de la réaction. Il a été acidifié pour amener le pH à 1. Après lavage, on a obtenu 993 g d'une solution dans le toluène d'un mélange de β-dicétones qu'on a analysé à 0,863 mole/kg, donnant un rendement de 85,7 % (sur la base de la cétone chargée).

Après élimination du solvant, un tel mélange réactionnel pouvaient être utilisé directement pour stabiliser des polymères halogénés (tels que le PVC).

Exemple 17: Palmitoylbenzoylméthane

L'appareil et la méthode étaient tels que décrits à l'exemple 1, mais avec les produits de départ suivants:

.    du méthylate de sodium préparé extemporanément        59,4 g (1,1 mole)
.    du xylène        715 ml.
.    du méthylpalmitate pur ($C_{15}H_{31}C(O)OMe$, PM 270,5)        324,6 g (1,2 mole)
.    de l'acétophénone pure        120,0 g (1,0 mole).

     (NB: le palmitoylbenzoylméthane, $C_{15}H_{31}COCH_2CO\Phi$, a un poids moléculaire de 358,6.)

Les opérations et la méthode étaient identiques à celles décrites à l'exemple 1. Le produit de distillation récupéré comprenait 150 g de xylène et 62 g de méthanol.

La phase xylène a été lavée et séchée, puis "dévolatilisée" (libérée de composants volatils) pour éliminer le xylène. (On a utilisé un évaporateur rotatif, sous pression réduite à 1500 Pa et à une température de 110°C à la fin de l'opération).

On a récupéré 405,6 g de produit brut sous la forme d'un solide jaune clair ayant un point de fusion de 56-60°C, contenant (par analyse CPG)

--    du palmitoylbenzoylméthane, PM 358,6        290,5 g (0,81 mole)
--    du dipalmitoylméthane, PM 492        16,3 g (0,033 mole)
--    du dibenzoylméthane, PM 224        9,0 g (0,04 mole).

Ainsi, le rendement global en β-dicétones était de 0,873 mole, soit 87,3 % (sur la base de la cétone chargée).

La teneur en β-dicétones du produit brut était de 77,9 %.

La recristallisation du produit brut à partir d'un mélange froid 50:50 d'acétone et de méthanol a conduit à la récupération de 292 g d'un produit blanc inodore ayant un point de fusion de 62-64°C, comprenant uniquement un mélange des deux β-dicétones suivantes:

--    le palmitoylbenzoylméthane        276 g (94,5 % de la matière froide)
--    le dipalmitoylméthane        16 g (5,5 % de la matière froide).

Le rendement de cristallisation était d'environ 95 %.

Exemple 18: Stéaroylbenzoylméthane ($C_{17}H_{35}COCH_2CO\Phi$, à partir de $C_{17}H_{35}C(O)OMe$):

On a procédé comme à l'exemple 1, en utilisant les produits de départ suivants:

.    du méthylate de sodium préparé extemporanément        59,4 g (1,1 mole)
.    du xylène        715 ml.
.    du méthylstéarate pur ($C_{17}H_{35}CO(O)Me$)        328,4 g (1,1 mole)
.    de l'acétophénone pure        120,05 g (1 mole).

414,5 g d'un produit brut sous la forme d'un solide jaune clair ayant un point de fusion de 58-65°C ont été obtenus. L'analyse par CPG a mis en évidence que ce produit contenait:

--    du stéaroylbenzoylméthane, PM 386,3        304 g (0,787 mole)
--    du distéaroylméthane, PM 548        13,7 g (0,025 mole)
--    du dibenzoylméthane, PM 224        7,9 g (0,035 mole)

     TOTAL de β-dicétones        325,6 g (0,847 mole)

Ainsi, le rendement chimique global en β-dicétones était de 84,7 % (0,847 mole).

La teneur en β-dicétones dans le produit brut obtenu était de 78,5 %.

La recristallisation de ce produit brut à partir d'un mélange froid 50:50 d'acétone et de méthanol a conduit à une récupération de 305 g d'un produit blanc inodore ayant un point de fusion de 67-69°C, comprenant uniquement un mélange des deux β-dicétones suivantes:

-- stéaroylbenzoylméthane     291,8 g (95,7 %)

-- dipalmitoylméthane     13,2 g (4,3 %).

Le rendement de la cristallisation était d'environ 96 %.

Remarque: La méthode décrite à l'exemple 10 du brevet américain 5.015.777, qui emploie un grand excès d'ester (4 fois théorie), n'a donné un rendement que de 45 % (d'un produit à 95 % pur). Nous n'avons utilisé qu'un excès de 10 % d'ester, et obtenu un rendement chimique de 84,7 % et un rendement après cristallisation d'environ 80 %.

Après élimination du solvant, un tel mélange réactionnel pouvait être mis en oeuvre directement pour stabiliser des polymères halogénés (tels que le PVC).

Exemple 19: Octanoylbenzoylméthane: Effet de la technique opératoire -- addition simultanée des deux réactants (ester et cétone):

Pour déterminer l'effet d'addition, au moins simultanément partielle, de la cétone et de l'ester, on a effectué les deux tests suivants, en utilisant l'appareil et le procédé décrits à l'exemple 1.

## Tableau:

## Type de test:

| Produits de départ (mole): | | | | | | Rendement: |
|---|---|---|---|---|---|---|
| Charges initiales: | | | | Additions supplémentaires (2): | | |
| | Ester (1) | MeONa | Xylène | $\Phi COCH_3$ | Ester | |
| A) Contrôle | 1,20 | 1,10 | 715 ml | 1 | ~~ | 89,5 % |
| B) Test avec addition simultanée | 0,200 | 1,10 | 715 ml | 1,00 | 1,00 | 79,7 % |

(1)    Méthyl octanoate.

(2)    Addition sur une période de 2 h.

     Test A:   Addition de $\Phi COCH_3$ seul.

     Test B:   Addition d'un mélange de $\Phi COCH_3$ et d'ester.

Les déterminations ont été effectuées par potentiométrie.

Le progrès par rapport à l'état de la technique est significatif. En effet, il est d'environ 10 % meilleur. Dans cette méthode d'opération, comme le montre le Test B, l'élimination continue de MeOH est réalisée, mais l'excès de l'ester est insuffisant (bien qu'une partie, 20 % de l'ester soit introduite dans la charge initiale). Par rapport au Test A, il n'est pas moins avantageux d'avoir au moins 30 % de l'ester que l'on doit faire réagir présent dans la charge initiale du réacteur.

Exemple 20: Isooctanoylbenzoylméthane

On a procédé comme à l'exemple 1, mais les réactants étaient les suivants:

.   du méthylate de sodium préparé extemporanément        62,6 g (1,16 mole)
.   du xylène        715 ml
.   du méthylissoactanoate, 99 % (1)        192 g (1,20 mole)
.   de l'acétophénone, 100 %        120 g (1,20 mole).

Le méthylisooctanoate était principalement constitué de 3,5-diméthylhexanoate de méthyle $CH_3CH(CH_3)CH_2CH(CH_3)C(O)OCH_3$.
On a analysé le produit à 625 meq d'ester/100 g.
Après acidification du mélange réactionnel par du $H_2SO_4$, lavage, etc., on a obtenu 941,4 g d'une solution dans le xylène.
Après élimination du xylène, 262 g d'un produit brut ont été obtenus sous la forme d'une huile jaune homogène.
On a déterminé par potentiométrie que ce produit contenait:

--   des fonctions β-dicétones        0,684 mole.
--   des fonctions acide carboxylique        0,327 mole.

Remarque: Le rendement chimique global en β-dicétones était de 68,4 %. Ceci est sensiblement inférieur à celui obtenu avec l'ester d'un acide linéaire en $C_8$ (c'est-à-dire 88,7 % -- voir exemple 7, dans les mêmes conditions). Ceci n'est pas étonnant car les esters ramifiés qui sont moins réactifs que les esters linéaires par rapport aux titres en $\Phi COCH_2$, produisent des sous-produits plus lourds.

Exemple 21: Hexanoyloctanoylméthane, $C_5H_{11}COCH_2COC_7H_{15}$

On a procédé comme à l'exemple 1, mais les réactants étaient les suivants:

.   du méthylate de sodium préparé extemporanément        59,4 g (1,10 mole)
.   du xylène        715 ml.
.   du méthyloctanoate (linéaire), 100 %        189,9 g (1,2 mole)
.   de la 2-heptanone, 100 %        114,2 g (1,0 mole).

Après acidification du mélange réactionnel par du $H_2SO_4$, lavage, etc., on a obtenu 1069,6 g d'une solution dans le xylène, d'une coloration orangée foncée, qu'on a déterminée par potentiométrie contenir: 0,771 mole de β-dicétones et 0,206 mole d'acides carboxyliques.
Le rendement chimique global en fonctions β-dicétone a ainsi été de 77,1 %. Ceci est sensiblement inférieur à celui obtenu en partant de méthyloctanoate et d'acétophénone, dans les mêmes conditions (par ex., l'exemple 11 a donné un rendement de 89,5 % dans des conditions très similaires).
Les méthylcétones aliphatiques, telles que la 2-heptanone, sont ainsi moins sélectives que l'acétophénone (elles ont une plus grande tendance à la crotonisation). L'analyse par CPG du mélange de β-dicétones a mis en évidence qu'il comprenait trois β-dicétones avec la distribution de poids suivante:

--   le dihexanoylméthane environ        3,5 %
--   l'hexanoyloctanoylméthane        92 %
--   le dioctanoylméthane        4,5 %.

Après élimination du solvant, un tel mélange réactionnel a été directement utilisable pour stabiliser des polymères halogénés (tels que le PVC).

Exemple 22: Octanoylbenzanoylméthane:

Effet de la méthode opératoire -- réaction sous pression réduite

L'objectif a été de déterminer l'influence de la pression et de vérifier la possibilité d'opérer sous pression réduite; on a procédé comme décrit à l'exemple 4.

Tableau:

| Type de test: Conditions opératoires: | | | | | Rendement: |
|---|---|---|---|---|---|
| | Durée d'addition | Température Φ | Solvant: | Pression (Torr): | |
| Témoin pression réduite | 2 h | 138°C | Xylène | 760 | 88,7 % |
| (8/30 × 10⁵ Pa) Pression réduite | 2 h | 95°C | Xylène | 200 | 91,6 % |
| (4/30 × 10⁵ Pa) Pression réduite | 2 h | 77°C | Xylène | 100 | 82,3 % |
| (4/30 × 10⁵ Pa) Pression réduite | 5 h | 77°C | Xylène | 100 | 89,6 % |

Ces tests montrent que la réaction peut être mise en oeuvre à basse température et à pression réduite. Toutefois, si la température est réduite à environ 80°C
ou inférieur, il est préférable d'ajouter la cétone à un taux d'addition réduit.

Bien que des modes de réalisation préférés de l'invention sont décrits ici en détail, l'homme de l'art comprendra que des variations peuvent y être apportées sans s'écarter de l'esprit de l'invention ou de la portée des revendications annexes.

**Revendications**

1. Méthode de préparation d'une β-dicétone de formule :

$$R_4\text{-CO-CHR}_5\text{-CO-R}_6$$

comprenant la réaction d'une cétone de formule $R_4$-CO-CHR$_5$H avec un ester de formule $R_6$-C(O)-OR$_7$, en présence d'un alcoolate dont l'alcool est volatil dans les conditions opératoires, dans laquelle ladite cétone est ajoutée audit ester tout en éliminant l'alcool progressivement au fur et à mesure de sa formation,
dans lesquelles :

-   $R_4$ et $R_6$, identiques ou différents, représentent chacun un groupement hydrocarboné et peuvent éventuellement être reliés pour former un composé cyclique ;
-   $R_5$ est un hydrogène ou un groupement hydrocarboné ;
-   $R_7$ est un groupement hydrocarboné tel que l'alcool $R_7$OH soit volatil dans les conditions réactionnelles ;

et dans laquelle le rapport molaire de ladite cétone audit ester est dans la gamme de 2/3:1 à 1:1.

2. Méthode selon la revendication 1, **caractérisée en ce que** la réaction se déroule à une température d'au moins 20°C supérieure au point d'ébullition dudit alcool.

3. Méthode selon l'une des revendications 1 ou 2, **caractérisée en ce que** la réaction se déroule à une température d'au moins 70°C, de préférence à une température allant de 110 à 150°C.

4. Méthode selon l'une des revendications 1 à 3, **caractérisée en ce que** le rapport molaire de ladite cétone audit ester est inférieur à 1.

5. Méthode selon l'une des revendications 1 à 4, **caractérisée en ce que** ledit ester est présent dans un excès jusqu'à 20 % de ladite cétone, et de préférence dans un excès de 10 à 20 % de ladite cétone.

6. Méthode selon l'une des revendications 1 à 5, **caractérisée en ce que** ledit ester a au moins un hydrogène dans une position alpha par rapport à la fonction ester et qu'il est au moins partiellement sous la forme du β-céto-ester.

7. Méthode selon l'une des revendications 1 à 6, **caractérisée en ce que** le rapport molaire de ladite cétone audit alcoolate est inférieur à 1.

8. Méthode selon la revendication 7, **caractérisée en ce que** le rapport molaire de ladite cétone audit alcoolate est dans la gamme de 2/3 à 1.

9. Méthode selon l'une des revendications 1 à 8, **caractérisée en ce que** ledit alcoolate est un alcool linéaire ou ramifié ayant de 1 à 6 atomes de carbone.

10. Méthode selon la revendication 9, **caractérisé en ce que** ledit alcoolate est un méthylate.

11. Méthode selon l'une des revendications 1 à 10, **caractérisée en ce que** ladite cétone est ajoutée à un taux d'au plus 0,001 mole de cétone par seconde par litre de suspension d'alcoolate dans le mélange réactionnel de départ, de préférence à un taux d'au plus 0,0002 mole/seconde/litre.

12. Méthode selon rune des revendications 1 à 11, **caractérisée en ce que** la réaction se déroule en présence d'un solvant.

13. Méthode selon la revendication 12, **caractérisée en ce que** ledit solvant a un point d'ébullition d'au moins 20°C au-dessus de la température de la réaction.

14. Méthode selon l'une des revendications 12 ou 13, **caractérisée en ce que** ledit solvant a un point d'ébullition, à pression atmosphérique, d'au moins 100°C. de préférence, allant de 130 à 200°C.

15. Méthode selon l'une des revendications 12 à 14, **caractérisée en ce que** ledit solvant a un point d'ébullition, à pression atmosphérique, d'au moins 20°C au-dessus du point d'ébullition dudit alcool.

16. Méthode selon l'une des revendications 12 à 15, **caractérisée en ce que** la réaction est effectuée au reflux du solvant, dans des conditions permettant l'élimination progressive dudit alcool au fur et à mesure de sa formation.

17. Méthode selon l'une des revendications 12 à 16, **caractérisée en ce que** ledit solvant est choisi parmi des hydrocarbures et des fractions hydrocarbonées.

18. Méthode selon la revendication 17, **caractérisé en ce que** ledit solvant est un hydrocarbure aromatique.

19. Méthode selon l'une des revendications 17 ou 18. **caractérisée en ce que** ledit solvant est choisi parmi les alkylbenzènes et les dialkylbenzènes.

20. Méthode selon l'une des revendications précédentes, **caractérisée en ce que** $R_4$ et $R_6$, identiques ou différents, représentent chacun un groupement hydrocarboné ayant de 1 à 30 atomes de carbone, de préférence de 1 à 18 atomes de carbone.

21. Méthode selon l'une des revendications précédentes, **caractérisée en ce que** $R_4$ et $R_6$, identiques ou différents, sont choisis parmi les radicaux alkyle ou alkényle, linéaires ou ramifiés, ayant de 1 à 24 atomes de carbone ; aralkyle ayant de 7 à 10 atomes de carbone ; et aryle ou cycloaliphatique, pouvant avoir une ou plusieurs liaisons doubles ayant chacun moins de 14 atomes de carbone ; lesdits radicaux peuvent éventuellement être substitués par des atomes d'halogène ou des groupements alkyles tels que méthyle et éthyle, et peuvent comprendre un ou plusieurs de -O-, -CO-O-, -CO-, et Si dans la chaîne aliphatique ; ou bien $R_4$ et $R_6$ peuvent ensemble former un groupement divalent ayant de 2 à 5 atomes de carbone, contenant éventuellement un hétéroatome tel que l'oxygène ou l'azote.

22. Méthode selon l'une des revendications précédentes, **caractérisée en ce que** $R_5$ est un atome d'hydrogène ou un groupement alkyle ayant de 1 à 4 atomes de carbone, pouvant être substitués ou non substitués et pouvant contenir un ou plusieurs de -O-, -CO-O-, -CO-.

**23.** Méthode selon l'une des revendications précédentes, **caractérisée en ce que** $R_5$ est un atome d'hydrogène.

**24.** Méthode selon l'une des revendications précédentes, **caractérisée en ce que** $R_7$ est un groupement alkyle ayant de 1 à 4 atomes de carbone, de préférence méthyle.

**Claims**

**1.** Method for preparing a β-diketone of formula:

$$R_4\text{-CO-CHR}_5\text{-CO-R}_6$$

comprising the reaction of a ketone of formula $R_4$-CO-CHR$_5$H with an ester of formula $R_6$-C(O)-OR$_7$ in the presence of an alcoholate whose alcohol is volatile under the operating conditions, in which the said ketone is added to the said ester while removing the alcohol progressively as it is formed,
in which formulae:

- $R_4$ and $R_6$, which are identical or different, each represent a hydrocarbon group and may be optionally linked in order to form a cyclic compound;
- $R_5$ is hydrogen or a hydrocarbon group;
- $R_7$ is a hydrocarbon group such that the alcohol $R_7$OH is volatile under the reaction conditions;

and in which the molar ratio of the said ketone to the said ester is in the range from 2/3:1 to 1:1.

**2.** Method according to Claim 1, **characterized in that** the reaction occurs at a temperature at least 20°C higher than the boiling point of the said alcohol.

**3.** Method according to either of Claims 1 and 2, **characterized in that** the reaction occurs at a temperature of at least 70°C, preferably at a temperature ranging from 110 to 150°C.

**4.** Method according to one of Claims 1 to 3, **characterized in that** the molar ratio of the said ketone to the said ester is less than 1.

**5.** Method according to one of Claims 1 to 4, **characterized in that** the said ester is present in an excess of up to 20% of the said ketone, and preferably in an excess of 10 to 20% of the said ketone.

**6.** Method according to one of Claims 1 to 5, **characterized in that** the said ester has at least one hydrogen in an alpha position with respect to the ester functional group and **in that** it is at least partially in the β-keto ester form.

**7.** Method according to one of Claims 1 to 6, **characterized in that** the molar ratio of the said ketone to the said alcoholate is less than 1.

**8.** Method according to Claim 7, **characterized in that** the molar ratio of the said ketone to the said alcoholate is in the range from 2/3 to 1.

**9.** Method according to one of Claims 1 to 8, **characterized in that** the said alcoholate is a linear or branched alcohol having from 1 to 6 carbon atoms.

**10.** Method according to Claim 9, **characterized in that** the said alcoholate is a methoxide.

**11.** Method according to one of Claims 1 to 10, **characterized in that** the said ketone is added at a rate of at most 0.001 mol of ketone per second per litre of alcoholate suspension to the starting reaction mixture, preferably at a rate of at most 0.0002 mol/second/litre.

**12.** Method according to one of Claims 1 to 11, **characterized in that** the reaction occurs in the presence of a solvent.

**13.** Method according to Claim 12, **characterized in that** the said solvent has a boiling point at least 20°C above the reaction temperature.

14. Method according to either of Claims 12 and 13, **characterized in that** the said solvent has a boiling point, at atmospheric pressure, of at least 100°C, preferably ranging from 130 to 200°C.

15. Method according to one of Claims 12 to 14, **characterized in that** the said solvent has a boiling point, at atmospheric pressure, at least 20°C above the boiling point of the said alcohol.

16. Method according to one of Claims 12 to 15, **characterized in that** the reaction is carried out at the reflux temperature of the solvent, under conditions allowing gradual removal of the said alcohol as it is formed.

17. Method according to one of Claims 12 to 16, **characterized in that** the said solvent is chosen from hydrocarbons and hydrocarbon fractions.

18. Method according to Claim 17, **characterized in that** the said solvent is an aromatic hydrocarbon.

19. Method according to either of Claims 17 and 18, **characterized in that** the said solvent is chosen from alkylbenzenes and dialkylbenzenes.

20. Method according to one of the preceding claims, **characterized in that** $R_4$ and $R_6$, which are identical or different, each represent a hydrocarbon group having from 1 to 30 carbon atoms, preferably from 1 to 18 carbon atoms.

21. Method according to one of the preceding claims, **characterized in that** $R_4$ and $R_6$, which are identical or different, are chosen from a linear or branched alkyl or alkenyl radical having from 1 to 24 carbon atoms; an aralkyl radical having from 7 to 10 carbon atoms; and an aryl or cycloaliphatic radical which may have one or more double bonds each having less than 14 carbon atoms; the said radicals may be optionally substituted with halogen atoms or alkyl groups such as methyl and ethyl groups, and may comprise one or more of -O-, -CO-O-, -CO-, and Si in the aliphatic chain; or alternatively $R_4$ and $R_6$ can together form a divalent group having from 2 to 5 carbon atoms, optionally containing a heteroatom such as oxygen or nitrogen.

22. Method according to one of the preceding claims, **characterized in that** $R_5$ is a hydrogen atom or an alkyl group having from 1 to 4 carbon atoms, which may be substituted or unsubstituted and which may contain one or more of -O-, -CO-O-, -CO-.

23. Method according to one of the preceding claims, **characterized in that** $R_5$ is a hydrogen atom.

24. Method according to one of the preceding claims, **characterized in that** $R_7$ is an alkyl group having from 1 to 4 carbon atoms, preferably a methyl group.

**Patentansprüche**

1. Verfahren zur Herstellung eines β-Diketons der Formel

$$R_4\text{-CO-CHR}_5\text{-CO-R}_6$$

wobei das Verfahren die Reaktion eines Ketons der Formel $R_4$-CO-CHR$_5$H mit einem Ester der Formel $R_6$-C(O)-OR$_7$ in Gegenwart eines Alkoholats, dessen Alkohol unter den Verfahrensbedingungen flüchtig ist, umfaßt, wobei das Keton zu dem Ester hinzugegeben wird und der Alkohol allmählich in dem Maße, wie er gebildet wird, entfernt wird,
wobei

- $R_4$ und $R_6$, identisch oder verschieden, jeweils eine Kohlenwasserstoffgruppe darstellen und gegebenenfalls miteinander verbunden sein können, um eine ringförmige Verbindung zu bilden;

- $R_5$ Wasserstoff oder eine Kohlenwasserstoffgruppe ist;

- R$_7$ eine solche Kohlenwasserstoffgruppe ist, daß der Alkohol R$_7$OH unter den Reaktionsbedingungen flüchtig ist;

wobei das Molverhältnis von Keton zu Ester im Bereich von 2/3 : 1 bis 1 : 1 liegt.

2. Verfahren nach Anspruch 1, **dadurch gekennzeichnet, daß** die Reaktion bei einer Temperatur abläuft, die mindestens 20 °C oberhalb der Siedetemperatur des Alkohols liegt.

3. Verfahren nach Anspruch 1 oder 2, **dadurch gekennzeichnet, daß** die Reaktion bei einer Temperatur von mindestens 70 °C, vorzugsweise bei einer Temperatur von 110 bis 150 °C, abläuft.

4. Verfahren nach einem der Ansprüche 1 bis 3, **dadurch gekennzeichnet, daß** das Molverhältnis von Keton zu Ester kleiner als 1 ist.

5. Verfahren nach einem der Ansprüche 1 bis 4, **dadurch gekennzeichnet, daß** der Ester in einem Überschuß von bis zu 20 % in bezug auf das Keton, vorzugsweise in einem Überschuß von 10 bis 20 % in bezug auf das Keton, vorhanden ist.

6. Verfahren nach einem der Ansprüche 1 bis 5, **dadurch gekennzeichnet, daß** der Ester mindestens ein Wasserstoffatom in einer α-Position in bezug auf die Esterfunktion aufweist und daß er mindestens teilweise in Form des β-Ketoesters vorliegt.

7. Verfahren nach einem der Ansprüche 1 bis 6, **dadurch gekennzeichnet, daß** das Molverhältnis von Keton zu Alkoholat kleiner als 1 ist.

8. Verfahren nach Anspruch 7, **dadurch gekennzeichnet, daß** das Molverhältnis von Keton zu Alkoholat im Bereich von 2/3 bis 1 liegt.

9. Verfahren nach einem der Ansprüche 1 bis 8, **dadurch gekennzeichnet, daß** das Alkoholat ein linearer oder verzweigter Alkohol mit 1 bis 6 Kohlenstoffatomen ist.

10. Verfahren nach Anspruch 9, **dadurch gekennzeichnet, daß** das Alkoholat ein Methylat ist.

11. Verfahren nach einem der Ansprüche 1 bis 10, **dadurch gekennzeichnet, daß** das Keton mit einem Durchsatz von höchstens 0,001 mol Keton pro Sekunde und pro Liter der Alkoholatsuspension in der Ausgangsreaktionsmischung, vorzugsweise mit einem Durchsatz von höchstens 0,0002 mol/Sekunde/Liter, zugegeben wird.

12. Verfahren nach einem der Ansprüche 1 bis 11, **dadurch gekennzeichnet, daß** die Reaktion in Gegenwart eines Lösemittels abläuft.

13. Verfahren nach Anspruch 12, **dadurch gekennzeichnet, daß** das Lösemittel einen Siedepunkt aufweist, der mindestens 20 °C oberhalb der Reaktionstemperatur liegt.

14. Verfahren nach Anspruch 12 oder 13, **dadurch gekennzeichnet, daß** das Lösemittel bei Atmosphärendruck einen Siedepunkt von mindestens 100 °C, vorzugsweise 130 bis 200 °C, aufweist.

15. Verfahren nach einem der Ansprüche 12 bis 14, **dadurch gekennzeichnet, daß** das Lösemittel einen Siedepunkt aufweist, der bei Atmosphärendruck mindestens 20 °C oberhalb des Siedepunkts des Alkohols liegt.

16. Verfahren nach einem der Ansprüche 12 bis 15, **dadurch gekennzeichnet, daß** die Reaktion unter Rückfluß des Lösemittels unter Bedingungen durchgeführt wird, welche die allmähliche Entfernung des Alkohols in dem Maße, wie er gebildet wird, ermöglichen.

17. Verfahren nach einem der Ansprüche 12 bis 16, **dadurch gekennzeichnet, daß** das Lösemittel aus Kohlenwasserstoffen und Kohlenwasserstofffraktionen ausgewählt wird.

18. Verfahren nach Anspruch 17, **dadurch gekennzeichnet, daß** das Lösemittel ein aromatischer Kohlenwasserstoff ist.

**19.** Verfahren nach Anspruch 17 oder 18, **dadurch gekennzeichnet, daß** das Lösemittel aus Alkylbenzolen und Dialkylbenzolen ausgewählt ist.

**20.** Verfahren nach einem der vorhergehenden Ansprüche, **dadurch gekennzeichnet, daß** $R_4$ und $R_6$, identisch oder verschieden, jeweils eine Kohlenwasserstoffgruppierung mit 1 bis 30 Kohlenstoffatomen, vorzugsweise 1 bis 18 Kohlenstoffatomen, darstellen.

**21.** Verfahren nach einem der vorhergehenden Ansprüche, **dadurch gekennzeichnet, daß** $R_4$ und $R_6$, identisch oder verschieden, ausgewählt sind aus linearen oder verzweigten Alkyl- oder Alkenylresten mit 1 bis 24 Kohlenstoffatomen; Aralkylresten mit 7 bis 10 Kohlenstoffatomen; und Aryl- oder cycloaliphatischen Resten, die eine oder mehrere Doppelbindungen mit jeweils weniger als 14 Kohlenstoffatomen aufweisen können; wobei diese Reste gegebenenfalls mit Halogenatomen oder Alkylgruppierungen, wie Methyl und Ethyl, substituiert sein können und ein oder mehrere Einheiten bzw. Gruppen -O-, -CO-O-, -CO- und Si in der aliphatischen Kette enthalten können; oder aber $R_4$ und $R_6$ zusammen eine zweiwertige Gruppierung mit 2 bis 5 Kohlenstoffatomen bilden können, die gegebenenfalls ein Heteroatom, wie Sauerstoff oder Stickstoff, enthalten kann.

**22.** Verfahren nach einem der vorhergehenden Ansprüche, **dadurch gekennzeichnet, daß** $R_5$ ein Wasserstoffatom oder eine Alkylgruppierung mit 1 bis 4 Kohlenstoffatomen ist, die substituiert sein kann oder nicht substituiert sein kann und ein oder mehrere Einheiten bzw. Gruppen -O-, -CO-O- und -CO- enthalten kann.

**23.** Verfahren nach einem der vorhergehenden Ansprüche, **dadurch gekennzeichnet, daß** $R_5$ ein Wasserstoffatom ist.

**24.** Verfahren nach einem der vorhergehenden Ansprüche, **dadurch gekennzeichnet, daß** $R_7$ eine Alkylgruppierung mit 1 bis 4 Kohlenstoffatomen, vorzugsweise Methyl, ist.